(19) Europäisches Patentamt
European Patent Office
Office européen des brevets

(11) EP 0 614 887 B1

(12) **EUROPEAN PATENT SPECIFICATION**

(45) Date of publication and mention of the grant of the patent:
**11.06.1997 Bulletin 1997/24**

(51) Int Cl.6: **C07D 209/08**, A61K 31/40

(21) Application number: **94301671.7**

(22) Date of filing: **09.03.1994**

(54) **Indole-sulfonamides as antitumor agents**

Indol-sulfonamide als Antitumormittel

Indole-sulfonamides comme agents antitumoraux

(84) Designated Contracting States:
**AT BE CH DE DK ES FR GB GR IE IT LI LU NL PT SE**
Designated Extension States:
**SI**

(30) Priority: **10.03.1993 US 29095**

(43) Date of publication of application:
**14.09.1994 Bulletin 1994/37**

(73) Proprietor: **ELI LILLY AND COMPANY**
**Indianapolis, Indiana 46285 (US)**

(72) Inventors:
- **Grindey, Gerald Burr**
  **Indianapolis, Indiana 46250 (US)**
- **Grossman, Cora Sue**
  **Indianapolis, Indiana 46250 (US)**
- **Howbert, James Jeffry**
  **Bellevue, Washington 98005 (US)**
- **Ray, James Edward**
  **Indianapolis, Indiana 46268 (US)**
- **Toth, John Eldon**
  **Indianapolis, Indiana 46278 (US)**

(74) Representative: **Tapping, Kenneth George et al**
**Lilly Industries Limited**
**European Patent Operations**
**Erl Wood Manor**
**Windlesham Surrey GU20 6PH (GB)**

(56) References cited:
**EP-A- 0 291 269**
**EP-A- 0 467 613**
**DE-B- 1 240 866**
**US-A- 5 116 874**
**US-A- 5 169 860**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 614 887 B1

## Description

In recent years fundamental advances have been made in the development of chemical agents and regimens of therapy to combat neoplastic diseases. Despite these continuing advances, cancers continue to exact intolerable levels of human pain and suffering. The need for new and better methods of treating neoplasms and leukemias continues to fuel efforts to find new classes of antitumor compounds, especially in the area of inoperable or metastatic solid tumors, such as the various forms of lung cancer. Of the one million new cases of cancer diagnosed in the United States each year, more than 90% represent non-hematopoetic tumors, where improvements in five-year survival rates have been modest, at best. B.E. Henderson, et al., Science, 254:1131-1137 (1991).

The recent avalanche of information regarding the basic biological processes involved in neoplasms has led to a deeper understanding of the heterogeneity of tumors. Ongoing work has led to the realization that individual tumors may contain many subpopulations of neoplastic cells that differ in crucial characteristics such as karyotype, morphology, immunogenicity, growth rate, capacity to metastasize, and response to antineoplastic agents.

It is because of this extreme heterogeneity among populations of neoplastic cells that new chemotherapeutic agents should have a wide spectrum of activity and a large therapeutic index. In addition, such agents must be chemically stable and compatible with other agents. It is also important that any chemotherapeutic regimen be as convenient and painless as possible to the patient.

This invention reports a series of novel sulfonylureas that are useful in the treatment of solid tumors. These compounds are orally active -- which, of course, results in less trauma to the patient -- and are relatively non-toxic. These compounds also have an excellent therapeutic index. The compounds and their formulations are novel.

Many sulfonylureas are known in the art.
Certain of these compounds are known to have hypoglycemic activities, and have been used medicinally as such agents. In addition, some sulfonylureas have been taught to have herbicidal and antimycotic activities. General reviews of compounds of this structural type are taught by Kurzer, Chemical Reviews, 50:1 (1952) and C.R. Kahn and Y. Shechter, Goodman and Gilman's, The Pharmacological Basis of Therapeutics, (Gilman, et al., 8th ed. 1990) 1484-1487.

Some diarylsulfonylureas have been reported as being active antitumor agents. e.g., U.S. Patent 5,169,860, of F. Mohamadi and M. Spees, issued December 8, 1992; U.S. Patent 4,845,128 of Harper, et al., issued July 4, 1989; U. S. Patent 5,110,830 of Harper, et al., issued May 5, 1992; U.S. Patent 5,116,874 of G.A. Poore, issued May 26, 1992; European Patent Publication 0467613 (published January 22, 1992); Grindey, et al., American Association of Cancer Research, 27:277 (1986); and Houghton, et al., Cancer Chemotherapy and Pharmacology, 25:84-88 (1989).

This invention provides the novel compounds of Formula I

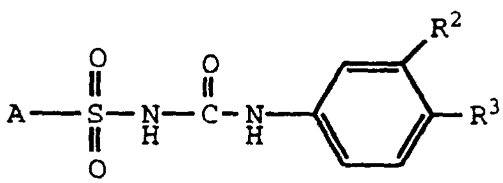

I

wherein:

A is

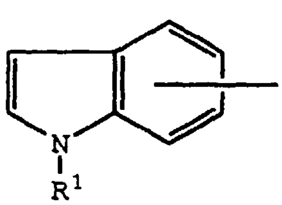

or

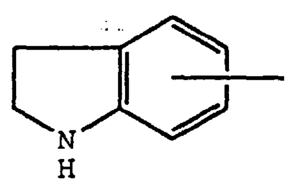

$R^1$ is $C_1$-$C_6$ alkyl;
$R^2$ and $R^3$ are independently selected from the group consisting of hydrogen, halo, $C_1$-$C_6$ alkyl, and trifluoromethyl, provided that no more than one of $R^2$ and $R^3$ can be hydrogen;

or a pharmaceutically acceptable salt or solvate thereof and the salts and solvates thereof. Such compounds are especially useful in the treatment of susceptible neoplasms in mammals.

As used herein, the term "halo" refers to fluoro, chloro, bromo, and iodo. The term "$C_1$-$C_6$ alkyl" refers to straight and branched chains of 1 to 6 carbon atoms and includes, but is not limited to, methyl, ethyl, propyl, isopropyl, butyl, isobutyl, t-butyl, pentyl, isopentyl, and hexyl.

Preferred compounds are those of Formula I in which $R^2$ and $R^3$ are independently selected from the group consisting of hydrogen, chloro, fluoro, bromo, iodo, methyl, ethyl, and trifluoromethyl.

The compounds of Formula I are generally referred to as derivatives of N-[[(substituted phenyl)amino]carbonyl]-2,3-dihydro-1H-indolesulfonamides or N-[[(substituted phenyl)amino]carbonyl]-($N_1$-substituted indole)sulfonamides. Alternatively, the compounds can be referred to as 1-(substituted phenyl)-3-($N_1$-substituted indolesulfonyl)ureas, or 1-(substituted phenyl)-3-(2,3-dihydro-1H-indolesulfonyl)ureas, or N- and N'-substituted sulfonylureas.

The compounds of Formula I can be prepared by methods known in the literature. Generally, these methods involve either the reaction of a sulfonamide with an isocyanate, or a reaction of a sulfonylcarbamate with an appropriately substituted aniline.

A preferred process for preparing a compound of Formula I comprises reacting an appropriately substituted sulfonamide of Formula II

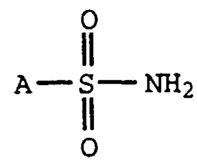

II

with an isocyanate of Formula III

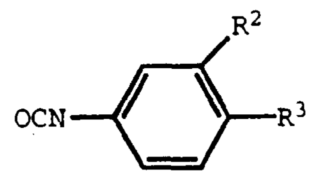

III

to provide the corresponding compound of Formula I.

The reaction is generally performed in a mixture of water and a water-miscible, non-reactive solvent such as tetrahydrofuran or acetone in the presence of a base such as sodium hydroxide, potassium hydroxide, lithium hydroxide, sodium methoxide, sodium hydride. Generally, an equimolar or slight molar excess of III is employed, although other ratios are operative. Usually, the amount of base used is approximately equimolar to the amount of II. The reaction is generally carried out from 0°C up to 100°C. At the preferred temperature of 20°C to 30°C, the reaction is usually complete within three hours.

An alternative preferred process for preparing a compound of Formula I involves reacting a sulfonamide of Formula II with an alkyl haloformate of the formula $XCOOR^4$, where X is bromo or chloro and $R^4$ is an alkyl group, preferably $C_1$-$C_3$ alkyl, to provide the carbamate of Formula IV

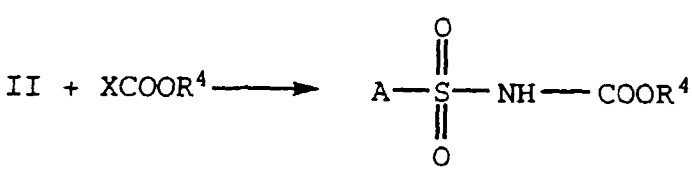

IV

The carbamate of Formula IV is then reacted with an aniline derivative of Formula V

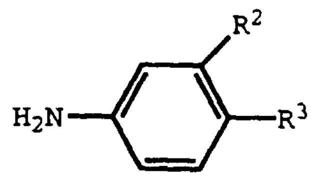

V

to provide the corresponding product of Formula I. The transformation of II into IV is usually accomplished in a non-reactive solvent, such as acetone or methyl ethyl ketone, in the presence of an acid scavenger, such as an alkali metal carbonate, for example potassium carbonate. A molar excess of the haloformate is usually added, although other ratios are operative. The reaction mixture is heated to a temperature from 30°C up to the reflux temperature of the mixture for a period of 1-6 hours to provide the desired intermediate IV.

Intermediate carbamate IV and the substituted aniline V are then heated together in an inert high-boiling solvent, such as dioxane, toluene, or diglyme, at temperatures from 50°C up to the reflux temperature of the mixture to provide the desired product of Formula I.

The carbamate of Formula IV can also be synthesized by the procedure described by Atkins and Burgess. G. Atkins and E. Burgess, Journal of the American Chemical Society, 94:6135 (1972). In this process triethylamine and a substituted aniline are mixed in the presence of a solvent such as benzene. To this mixture a sulfamoyl chloride is added to produce the carbamate of Formula IV.

Intermediates II, III, and V and any other reagents required for these methods of preparation are commercially available or can be prepared by methods known in the art.

This invention includes methods employing the pharmaceutically acceptable salts of the Formula I compounds, and also includes the pharmaceutically acceptable salts of the Formula I compounds. The Formula I compounds can react with basic materials such as alkali-metal or alkaline-earth-metal hydroxides, carbonates, and bicarbonates including, without limitation, sodium hydroxide, sodium carbonate, potassium hydroxide, calcium hydroxide, lithium hydroxide, to form pharmaceutically acceptable salts such as the corresponding sodium, potassium, lithium, or calcium salt. Organic bases can also be used, including primary, secondary, and tertiary alkyl amines such as methylamine, triethylamine.

This invention further encompasses the pharmaceutically acceptable solvates of the compounds of Formula I. The Formula I compounds can combine with solvents such as water, methanol, ethanol and acetonitrile to form pharmaceutically acceptable solvates such as the corresponding hydrate, methanolate, ethanolate and acetonitrilate.

The terms and abbreviations used in the instant examples have their normal meanings unless otherwise designated. For example "°C" refers to degrees Celsius; "N" refers to normal or normality; "mmole" refers to millimole or millimoles; "g" refers to gram or grams; "ml" means milliliter or milliliters; "M" refers to molar or molarity; "FDMS" refers to field desorption mass spectrometry; "EIMS" refers to electron ionization mass spectrometry; and "NMR" refers to nuclear magnetic resonance.

The following examples further illustrate the preparation of the compounds of Formula I. These examples are illustrative only and are not intended to limit the scope of the invention in any way.

Example 1

N-[[(4-chlorophenyl)amino]carbonyl]-2,3-dihydro-1H-indole-5-sulfonamide

N-acetyl indoline (10 g, 0.062 mole) was dissolved in 30 ml of methylene chloride and cooled to 0°C. Chlorosulfonic acid (28.9 g, 0.248 mole) was added dropwise over 15 minutes. The reaction mixture was then warmed to 40°C and maintained at that temperature for about two hours.

After the two hours, the reaction mixture was cooled to room temperature and carefully poured onto 300 ml of ice. Methylene chloride (50 ml) and tetrahydrofuran (10 ml) were then added. The organic layer was then dripped through sodium sulfate. Two further extractions were taken using 50 ml of methylene chloride each, and dripped through sodium sulfate. The three organic layers were combined and evaporated to a solid.

The resulting solid was re-dissolved in the smallest possible quantity of acetone and poured into 200 ml of concentrated ammonium hydroxide. The resulting solution was warmed to 60°C, then diluted with 400 ml of water. This solution was cooled to room temperature, and then allowed to sit at room temperature for several hours.

The resulting solid, comprising N-acetyl indoline-5-sulfonamide, was collected by filtration, washed with water, and dried in vacuo. Yield: 8.01 g (54%).

The N-acetyl indoline-5-sulfonamide (7.2 g, 0.030 mole) was suspended in acetone (16 ml) and 31.5 ml of 1 N sodium hydroxide (0.0315 mole) were added. Within 5 minutes, a clear, slightly yellow solution had formed, which was stirred at room temperature for 15 minutes.

4-Chlorophenylisocyanate (5.07 g, 0.033 mole) was dissolved in 16 ml of acetone, and then was added dropwise to the solution containing the N-acetyl indoline-5-sulfonamide. This mixture was stirred overnight at room temperature. A solid which had formed was removed by filtration, and the filtrate was acidified with 31.5 ml of 1N hydrochloric acid, causing the formation of a gummy white precipitate. This mixture was diluted with 150 ml of water and stirred overnight at room temperature. The resulting product, N-[[(4-chlorophenyl)amino]carbonyl]-1-acetyl-2,3-dihydro-1H-indole-5-sulfonamide, was collected by filtration, washed with water, and dried in vacuo.
Yield: 10.64 g (90%).

The N-[[(4-chlorophenyl)amino]carbonyl]-1-acetyl-2,3-dihydro-1H-indole-5-sulfonamide (0.79 g, 0.002 mole) was deacetylated by the addition of hydrazine hydrate (0.01 mole). This mixture was warmed to 70°C and maintained at this temperature for 5 hours. The mixture was then cooled to room temperature and evaporated to an oil. The oil was taken up in water and extracted with an ethyl acetate/tetrahydrofuran mixture. The organic layer was dripped through sodium sulfate and evaporated to an oil. The title product was isolated by flash chromatography (3:1 ether:hexane plus 1% acetic acid) as 0.17 g solid (24%).

Analysis of the product gave the following results: $^1$H NMR (300 MHz, $d_6$-DMSO) $\delta$ 2.99 (t, J=8 Hz, 2H, $CH_2C\underline{H}_2$), 3.55 (t, J=8 Hz, 2H, $CH_2C\underline{H}_2$), 6.49 (d, J=8 Hz, 1H, Ar$\underline{H}$), 6.55 (brs. 1H, $CH_2N\underline{H}$), 7.34 (ABq, J=8 Hz, $\Delta\upsilon$=21 Hz, 2H, Ar$\underline{H}$), 7.51 (s overlapping d, 2H, Ar$\underline{H}$), 8.80 (s, 1H, ArN$\underline{H}$), 10.40 (brs, 1H, $SO_2N\underline{H}$); UV(EtOH) $\lambda_{max}(\varepsilon)$ 252 (24510) nm; IR(KBr) 3340, 1607, 1541, 1494, 1450, 1170, 1145, 1117, 1065, 576, and 550 $cm^{-1}$; EIMS *m/e* 351 ($M^+$)

| Analysis for $C_{15}H_{14}ClN_3O_3S$: | | | | |
|---|---|---|---|---|
| Theory: | C, 51.21; | H, 4.01; | N, 11.94; | S, 9.11. |
| Found: | C, 51.41; | H, 4.19; | N, 11.70; | S, 8.98. |

Example 2

N-[[(3,4-dichlorophenyl)amino]carbonyl]-2,3-dihydro-1H-indole-5-sulfonamide

Preparation of ethyl indoline-5-sulfonamide-1-carboxylate

To a flask containing chlorosulfonic acid (125 ml, 1.9 moles) was added ethyl indoline-1-carboxylate (70.5 g, 0.37 mole) in portions under nitrogen purge with vigorous stirring over 20 minutes. The ethyl indoline-1-carboxylate was prepared using procedures known in the art. See, e.g., B. de Oliveira, et al., Journal of the Chemical Scoiety, Perkins Transactions I, 1977:1477 (1977). After 90 minutes at room temperature the reaction mixture was carefully poured onto 500 g crushed ice and extracted with methylene chloride (3 x 200 ml). The combined organic extracts were dried by filtration through calcium sulfate and evaporated. The resulting crude sulfonyl chloride was stirred with concentrated ammonium hydroxide (500 ml) for 2 hours. Filtration was followed by washing with water (500 ml), which was followed by washing with diethyl ether (500 ml) and vacuum drying to give 80.4 g (81%) of the product sulfonamide as a white solid.

Analysis of the product gave the following results: mp=164-165°C; $R_f$(1/1, EtOAc/hexane)=0.28 ; $^1$H NMR (300 MHz, $d_6$-DMSO) $\delta$ 1.26 (t, 3H, J=7.1 Hz, $CH_2C\underline{H}_3$), 3.13 (t, 2H, J=8.7 Hz, $CH_2C\underline{H}_2$), 3.97 (t, 2H, J=8.7 Hz, $CH_2C\underline{H}_2$), 4.19 (q, 2H, J=7.1 Hz, $C\underline{H}_2CH_3$), 7.18 (s, 2H, exchanges with $D_2O$, $SO_2N\underline{H}_2$), 7.61-7.63 (s overlapping with d, 2H, Ar-$\underline{H}$) and 7.70 (bs, 1H, Ar-$\underline{H}$); UV(EtOH) $\lambda_{max}(\varepsilon)$ 262.6 (20668), 208,6 (20726) and 204.6 (20527) nm; IR(KBr) 3326, 3229, 1693, 1489, 1325, 1186, 1046, 911, 828 and 768 $cm^{-1}$;
FDMS(MeOH) *m/e* 270 ($M^+$).

| Analysis for $C_{11}H_{14}N_2O_4S$: | | | |
|---|---|---|---|
| Theory: | C, 48.88; | H, 5.22; | N, 10.36. |
| Found: | C, 49.08; | H, 5.40; | N, 10.56. |

To a solution of ethyl indoline-5-sulfonamide-1-carboxylate (12.0 g, 44.0 mmoles) in 1N sodium hydroxide solution (44 ml) and acetone (22 ml), was added, dropwise, a solution of 3,4-dichlorophenylisocyanate (8.6 g, 44.3 mmoles) in acetone (22 ml) over 10 minutes. Two hours later the mixture was filtered and the filtrate treated with 1N hydrochloric acid solution (44 ml). The resulting solid was collected by filtration and rinsed with water (100 ml) and then slurried in ethanol (250ml) for 1 hour. Filtration was followed by washing with ethanol (50 ml) which was followed by washing with

diethyl ether (200 ml) and vacuum drying to give the purified N-[[(3,4-dichlorophenyl)amino]carbonyl]-2,3-dihydro-1-ethylcarboxylate-1H-indole-5-sulfonamide. Yield: 17.8 g (89%).

Analysis of the product gave the following results: mp=187-188°C; $R_f$(9/1, $CHCl_3$/MeOH)=0.35 ; $^1$H NMR (300 MHz, $d_6$-DMSO) δ 1.26 (t, 3H, J=7.0 Hz, $OCH_2C\underline{H}_3$), 3.15 (t, 2H, J=8.4 Hz, $CH_2C\underline{H}_2$), 4.00 (t, 2H, J=8.4 Hz, $CH_2C\underline{H}_2$), 4.19(q, 2H, J=7.0 Hz, $OC\underline{H}_2CH_3$), 7.27 (m, 1H, Ar-H), 7.50 (d, 1H, J=8.8 Hz, Ar-H), 7.68 (s, 1H, Ar-H), 7.73-7.85 (m overlapping s, 3H, Ar-H), 9.10 (s, 1H, exchanges with $D_2O$, NH) and 10.9 (bs, 1H, exchanges with $D_2O$, $SO_2N\underline{H}$); IR (KBr) 3350, 1732, 1677,1595,1540, 1449, 1320, 1197 1050 and 889 $cm^{-1}$; UV(EtOH) $\lambda_{max}(\varepsilon)$ 265.0 (19475), 252.4 (20830) and 208.8 (34048) nm; FDMS (MeOH) m/e 457, 459, 461 ($M^+$).

| Analysis for $C_{18}H_{17}Cl_2N_3O_5S$: | | | |
|---|---|---|---|
| Theory: | C, 47.17; | H, 3.74; | N, 9.17. |
| Found: | C, 47.37; | H, 3.91; | N, 9.02. |

The carbamate prepared supra (11.0 g, 25.0 mmol) was heated at reflux in a 2N potassium hydroxide solution (125 ml) for 1 hour. After cooling in an ice bath, the reaction mixture was quenched by the addition of 5N hydrochloric acid (50 ml). Filtration and drying gave the crude title product, which was purified by dissolution in 1N sodium hydroxide (50 ml) and water (450 ml), followed by filtration to remove the insoluble material. Treatment of the filtrate with 1N hydrochloric acid (50 ml) precipitated a solid which was collected by filtration and dried to give 3.9 g (40%) of N-[[(3,4-dichlorophenyl)amino]carbonyl]-2,3-dihydro-1H-indole-5-sulfonamide.

Analysis of the product gave the following results: mp=125°C; $R_f$ (THF)=0.52; $^1$H NMR (300 MHz, $d_6$-DMSO) δ 2.96 (t, 2H, J=8.4 Hz, $CH_2C\underline{H}_2$), 3.52 (t, 2H, J=8.4 Hz, $CH_2C\underline{H}_2$), 6.45 (d, 1H, J=8.1 Hz, Ar-H), 6.55(bs, 1H, exchanges with $D_2O$, NH), 7.25 (m, 1H, Ar-H), 7.46 (d overlapping s, 3H, Ar-H), 7.67 (m, 1H, Ar-H), 9.02 (s, 1H, exchanges with $D_2O$, NH) and 10.6 (bs, 1H, exchanges with $D_2O$, $SO_2N\underline{H}$); UV(1:1, pH=7 buffer/MeOH) $\lambda_{max}(\varepsilon)$ 258.2 (30477) and 209.6 (43326) nm; IR(KBr) 3344, 1707, 1607, 2477, 1171, 1119 and 857 $cm^{-1}$; FDMS (MeOH) *m/e* 385, 387, 389 ($M^+$).

| Analysis for $C_{15}H_{13}Cl_2N_3O_3S$: | | | |
|---|---|---|---|
| Theory: | C, 46.64; | H, 3.39; | N, 10.80. |
| Found: | C, 46.68; | H, 3.40; | N, 10.81. |

Example 3

N-[[(3,4-dichlorophenyl)amino]carbonyl]-1-methyl-1H-indole-5-sulfonamide

N-methyl-indoline-5-sulfonamide

A 3-liter, 3-neck flask with mechanical stirrer and nitrogen purge line was charged with ethyl-indoline-1-carboxylate-5-sulfonamide (27 g, 100 mmoles), as prepared in Example 2, and 1000 ml of anhydrous tetrahydrofuran. Under nitrogen purge was then added lithium aluminum hydride (95%, 10 g, 250 mmoles) in portions over 20 minutes, resulting in strong exotherms. The reaction was stirred at room temperature and monitored using HPLC (reverse-phase, 40/60/0.2% acetonitrile/water/phosphoric acid, 1 ml/min, monitoring at 254nm). After 2 hours the mixture was cooled in an ice-bath and carefully quenched by the addition of ice until no further reaction was noted. Concentrated hydrochloric acid (65 ml) was next added until the pH equaled 3. The inorganic solids were removed by filtration and the filtrate evaporated to give a tan solid (23 g). Purification was effected by slurrying the crude solid in 250 ml of $H_2O$ for 30 minutes and filtering, followed by rinsing of the cake with $H_2O$ (300 ml) and diethyl ether (300 ml). Vacuum drying gave 17.3 g (81%) of the product sulfonamide. Recrystallization from methanol gave an analytical sample.

Analysis of the product gave the following results: mp=176-177°C; $R_f$(1/1 EtOAc/hexane) = 0.29 ; $^1$H NMR (300 MHz, $d_6$-DMSO) δ 2.74 (s 3H, $NC\underline{H}_3$), 2.92 (t, 2H, J = 8.4 Hz, $CH_2C\underline{H}_2$), 3.38 (t, 2H, J = 8.4 Hz, $CH_2C\underline{H}_2$), 6.47 (d, 1H, J=8.3 Hz, Ar-H), 6.91 (bs, 2H, exchanges with $D_2O$, $SO_2N\underline{H}_2$), 7.39 (s, 1H, Ar-H) and 7.44 (d, 1H, J=8.3 Hz, Ar-H); IR (KBr)3314, 3239, 1605, 1509, 1313, 1170 and 1062 $cm^{-1}$; FDMS(MeOH) *m/e* 212 ($M^+$).

Analysis for $C_9H_{12}N_2O_2S$:

| | | | |
|---|---|---|---|
| Theory: | C, 50.92; | H, 5.70; | N, 13.20. |
| Found: | C, 50.87; | H, 5.62; | N, 12.91. |

The N-methyl-indoline-5-sulfonamide (225 mg, 1.06 mmol) was mixed with 10% palladium on carbon (84 mg) in methanol (8 ml) and these were refluxed for 23 hours. Additional 10% palladium on carbon (100 mg) and methanol (5 mL) were added, and refluxing continued for an additional 23 hours. The cooled reaction mixture was filtered through a Celite® pad, evaporated, and the crude solid redissolved in tetrahydrofuran and again filtered through a Celite® pad. Evaporation of the filtrate gave 211 mg (95%) of N-methyl-1H-indole-5-sulfonamide. This intermediate could also be produced from the reaction of N-methyl-indoline-5-sulfonamide and 2,3-dichloro-5,6-dicyano-1,4-benzoquinone (DDQ) in refluxing ethylene glycol monomethyl ether in 35% yield using known procedures. See, e.g., H. Breuer and H. Höhn, Chimie Therapeutique, 6:659 (1973).

Analysis of the product gave the following results: mp=225°-227°C; $R_f$ (EtOAc)=0.59; $^1H$ NMR (300 MHz, $d_6$-DMSO) δ 3.82 (s, 3H, N-C$\underline{H}_3$), 6.58 (d, 1H, J=3.0 Hz, Ar-$\underline{H}$), 7.11 (s, 2H, exchanges with $D_2O$, $SO_2N\underline{H}$), 7.47 (d, 1H, J=3.0 Hz, Ar-$\underline{H}$), 7.58 (s, 2H, Ar-$\underline{H}$) and 8.03 (s, 1H, Ar-$\underline{H}$); IR(KBr) 3330, 3235, 2948, 1512, 1326, 1145, and 1062 $cm^{-1}$; FDMS (MeOH) *m/e* 210 ($M^+$).

| Analysis for $C_9H_{10}N_2O_2S \cdot 0.33$ THF: | | | |
|---|---|---|---|
| Theory: | C, 52.96; | H, 5.44; | N, 11.97. |
| Found: | C, 52.62; | H, 5.06; | N, 11.53. |

The 1-methyl-1H-indole-5-sulfonamide was then reacted with 3,4-dichlorophenylisocyanate (1.16 g, 6.20 mmoles) as described in Example 2. This reaction resulted in a yield of 620 mg of N-[[(3,4-dichlorophenyl)amino]carbonyl]-1-methyl-1H-indole-5-sulfonamide.

Physical chemistry of the product of this example was consistent with the physical characteristics of the title product as exemplified in Example 4, infra.

## Example 4

Alternative procedure for synthesis of N-[[(3,4-dichlorophenyl)amino]carbonyl]-1-methyl-1H-indole-5-sulfonamide

An aliquot of [(2,3-dihydro-1-methyl-1H-indol-5-yl)sulfonyl]carbamic acid, ethyl ester was prepared by procedures known in the art. See, e.g., Burgess and Atkins, supra. N-Methylindoline, prepared as described in G. Gribble, et al., Synthesis, 1977:859 (1977), was reacted with carbethoxysulfamoyl chloride (28 g, 150 mmoles) and triethylamine (21 ml, 150 mmoles) in 200 ml benzene. Crude yield was 25 g (59%) as a mixture of regioisomers. Chromatography (silica gel, 100% $CH_2Cl_2$) gave the desired [(2,3-dihydro-1-methyl-1H-indol-5-yl)sulfonyl]carbamic acid, ethyl ester. Yield: 11.3 g (27%).

Analysis of the product gave the following results: $R_f$ (1/1 EtOAc/hexane)=0.36 ; $^1H$ NMR (300 MHz, $d_6$-DMSO) δ 1.06 (t 3H, J=7.0 Hz, $OCH_2C\underline{H}_3$), 2.80 (s, 3H, $NC\underline{H}_3$), 2.90 (t, 2H, J=8.5 Hz, $NCH_2C\underline{H}_2$), 3.45 (t, 2H, J=8.5 Hz, $NC\underline{H}_2CH_2$), 3.96 (q, 2H, J=7.0 Hz, $OC\underline{H}_2CH_3$), 6.50 (d, 1H, J=8.4 Hz, Ar-$\underline{H}$) , 7.38 (s, 1H, Ar-$\underline{H}$), 7.50 (d, 1H, J=8.4 Hz, Ar-$\underline{H}$) and 11.5 (bs, 1H, exchanges with $D_2O$, N$\underline{H}$); FDMS (MeOH) *m/e* 284 ($M^+$).

| Analysis for $C_{12}H_{16}N_2O_4S$: | | | |
|---|---|---|---|
| Theory: | C, 50.69; | H, 5.67; | N, 9.85. |
| Found: | C, 50.94; | H, 5.74; | N, 9.85. |

A solution of the (2,3-dihydro-1-methyl-1H-indole-5-sulfonyl)carbamic acid, ethyl ester produced supra (2.0 g, 7.0 mmoles) in acetic acid (40 ml) was treated with manganese (III) acetate dihydrate (2.55 g, 9.5 mmoles) and stirred at room temperature for 15 hours. The reaction mixture was poured into water (400 ml) and the resulting solid collected by filtration. The solid was resuspended in water, collected by filtration and air dried to give 1.5 g (75%) of (1-methyl-1H-indol-5-yl-sulfonyl)carbamic acid, ethyl ester. An analytical sample was obtained by silica gel flash chromatography (ethyl acetate/hexane).

Analysis of the product gave the following results: mp=153°-155°C; $R_f$ (EtOAc)=0.56; $^1H$ NMR (300 MHz, $d_6$-DMSO) δ 1.05 (t, 3H, J=7.2 Hz, $CH_2C\underline{H}_3$), 3.83 (s, 3H, N-C$\underline{H}_3$), 3.93 (q, 2H, J=7.2 Hz, $C\underline{H}_2CH_3$), 6.65 (d, 1H, J=3.1 Hz, Ar-$\underline{H}$), 7.53 (d, 1H, J=3.1 Hz, Ar-$\underline{H}$), 7.62 (s, 2H, Ar-$\underline{H}$), 8.15 (s, 1H, Ar-$\underline{H}$) and 11.70 (bs, 1H, exchanges with $D_2O$,

$SO_2N\underline{H}$); UV(EtOH) $\lambda_{max}(\varepsilon)$ 284.5 (4494) and 234.0 (41100) nm; IR(KBr) 3236, 1749, 1433, 1342, 1223, 1143 and 1058 $cm^{-1}$; FDMS (MeOH) *m/e* 282 ($M^+$).

| Analysis for $C_{12}H_{14}N_2O_4S$: | | | |
|---|---|---|---|
| Theory: | C, 51.05; | H, 5.00; | N, 9.92. |
| Found: | C, 51.20; | H, 4.71; | N, 9.97. |

The carbamate produced supra (1.0 g, 3.55 mmoles) was then reacted with 3,4-dichloroaniline (0.69 g, 4.26 mmoles) in refluxing toluene (35 ml) for 2 hours with periodic removal of distillate from a Dean-Stark trap over this period (10 ml total). The title product (1.19 g, 85%) was collected from the cooled reaction mixture by filtration and air-drying.

Analysis of the product gave the following results: mp=193°-194°C; $R_f$ (19/1, $CH_2Cl_2$/MeOH)=0.35 ; $^1H$ NMR (300 MHz, $d_6$-DMSO) $\delta$ 3.83 (s, 3H, N-$C\underline{H}_3$), 6.65 (d, 1H, J=3.0 Hz, Ar-$\underline{H}$), 7.22 (dd, 1H, J=2.5, 8.8 Hz, Ar-$\underline{H}$), 7.45 (d, 1H, J=8.8 Hz, Ar-$\underline{H}$), 7.52 (d, 1H, J=3.1 Hz, Ar-$\underline{H}$), 7.61-7.71 (overlapping multiplets, 3H, Ar-$\underline{H}$), 8.20 (s, 1H, Ar-$\underline{H}$), 9.04 (s, 1H, exchanges with $D_2O$, $N\underline{H}$), and 10.81 (bs, 1H, exchanges with $D_2O$, $SO_2N\underline{H}$); UV(EtOH) $\lambda_{max}(\varepsilon)$ 284.8 (6944), 235.0 (58699) and 210.2 (41775) nm; IR(KBr) 3330, 3235, 1707, 1588, 1526, 1462, 1324, 1149 and 1042 $cm^{-1}$; FDMS (MeOH) *m/e* 397, 399, 401 ($M^+$).

| Analysis for $C_{16}H_{13}Cl_2N_3O_3S$: | | | |
|---|---|---|---|
| Theory: | C, 48.25; | H, 3.29; | N, 10.55. |
| Found: | C, 48.50; | H, 3.41; | N, 10.47. |

In another embodiment this invention provides a method of treating a susceptible neoplasm in a mammal which comprises administering to a mammal in need of said treatment an effective amount for treating a susceptible neoplasm of a compound of Formula I:

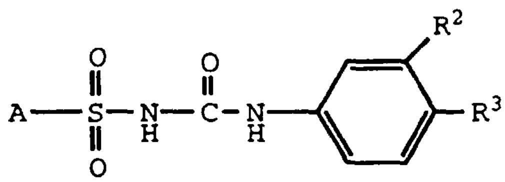

I

wherein:

A is

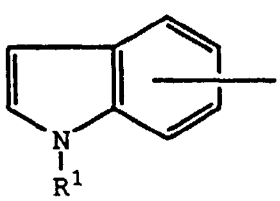

or

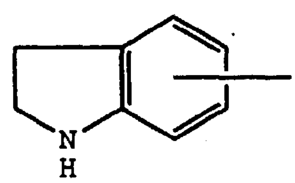

$R^1$ is $C_1$-$C_6$ alkyl;

$R^2$ and $R^3$ are independently selected from the group consisting of hydrogen, halo, $C_1$-$C_6$ alkyl, and trifluoromethyl, provided that no more than one of $R^2$ and $R^3$ can be hydrogen;

or a pharmaceutically acceptable salt or solvate thereof.

The compounds of Formula I have been shown to be active against transplanted mammalian tumors in vivo. To demonstrate the anti-tumor activity of the compounds of Formula I, some of these compounds were tested in mice bearing different allograft and xenograft tumors.

One of the tumor models used for showing the anti-neoplastic activity of the sulfonylureas of this invention was the human colon xenograft VRC5. J.A. Houghton and D.M. Taylor, British Journal of Cancer, 37:213-223 (1978). This tumor was obtained from St. Jude's Children's Research Hospital and has been widely used as a human tumor model.

Another tumor model employed C3H mice bearing the widely used allograft 6C3HED lymphosarcoma, also known as the Gardner lymphosarcoma (GLS). The 6C3HED lymphosarcoma was obtained from the Division of Cancer Treatment, National Cancer Institute, Tumor Bank, maintained at E. G. and G. Mason Research (Worcester, Massachusetts).

First passage tumors were stored in liquid nitrogen, using standard techniques. The transplanted tumor was reestablished from the Tumor Bank every six months or as needed. The tumor was maintained by serial passage twice weekly in the host mice.

In the procedures utilized here, the tumor was removed from passage animals and minced into cubic fragments having 1 to 3 mm edges using sterile techniques. Tumor pieces were checked for sterility using both Antibiotic Medium 1 and Brain Heart Infusion (Difco, Detroit, Michigan). The xenograft tumor pieces were implanted into the recipient CD1 Nu/Nu mice subcutaneously in an axillary site by trochar. The allograft 6C3HED tumor pieces were implanted into the recipient C3H mice in an analogous fashion.

Drug therapy on the appropriate schedule was initiated seven days after tumor implantation for the mice bearing the xenograft tumors, one day after implantation for the mice bearing the 6C3HED lymphosarcoma. The compound being tested was mixed with 2.5% Emulphor EL620 from GAF Corporation (1:40 dilution in 0.9% saline). The total dosage volume for each administration was 0.5 ml. All animals were weighed at the beginning and end of administration of the subject compounds. Food and water were provided ad libitum.

Each control group and each dosage level of the treated groups consisted of of 9 or 10 mice selected at random from the pool of implanted animals. The formulations were administered orally by gavage with the use of an 18-gauge needle. Compounds were dosed daily for 10 days for the studies using the human tumor xenografts and 8 days for the studies using the allograft.

The tumor was measured five days after treatment ended with two dimensional measurements (width and length) of the tumor taken using digital electronic calipers interfaced to a microcomputer. J.F. Worzalla, et al., Investigational New Drugs, 8:241-251 (1990). Tumor weights were calculated from these measurements using the following formula:

$$\text{Tumor weight (mg)} = \frac{\text{tumor length (mm) x [tumor width (mm)]}^2}{2}$$

At least one control group of an equal number of mice was treated with the same volume of 2.5% Emulphor only. The percent inhibition is determined by subtracting the ratio of the mean tumor size of the test group relative to the control group from one and multiplying the result by 100.

The results of several experiments in mice bearing the VRC5 human colon adenocarcinomas, and the 6C3HED lymphosarcoma when the Formula I compounds were administered orally are provided in Table I. In the table, Column 1 refers to the example number of the compound tested; Column 2 describes the particular human tumor xenograft or mouse allograft being studied; Column 3 gives the dosage level of the compound of Formula I in milligrams per kilogram of body weight; Column 4 describes the percent inhibition of tumor growth; and Column 5 tallies the number of mice which died during the course of the experiment relative to the total number of animals in the group.

Table I

Activity of the Compounds of Formula I Against Allograft and Xenograft Tumors In Vivo

| Example No. | Tumor | Dosage (mg/kg) | Percent Inhibition | Toxic/Total |
|---|---|---|---|---|
| 1 | 6C3HED | 280 | 44 | 0/9 |
| 2 | 6C3HED | 900 | 100 | 1/10 |
| | | 450 | 100 | 0/10 |
| | VRC5 | 40 | 56 | 0/9 |
| | | 20 | 18 | 0/9 |
| 3 | VRC5 | 80 | 100 | 0/10 |
| | | 40 | 95 | 1/10 |
| | | 20 | 81 | 1/9 |

The compounds of Formula I are also active against other tumor models, in addition to the ones shown above, including several which are usually considered to be refractory to chemotherapy. These compounds demonstrate an expansive range of antineoplastic activity with minimal toxicity.

Since the compounds of Formula I are antineoplastic agents, the invention also provides a method of treating a susceptible neoplasm in a mammal which comprises administering to a mammal in need of said treatment an effective amount of a compound of Formula I or a pharmaceutically acceptable salt or solvate thereof. In particular, the present compound is useful in treating solid tumors including carcinomas such as ovarian, non-small cell lung, gastric, pancreatic, prostate, renal cell, breast, colorectal, small cell lung, melanoma, and head and neck; and sarcomas such as Kaposi's sarcoma and rhabdomyosarcoma.

The compounds of Formula I are usually administered in the form of pharmaceutical compositions. These compositions can be administered by a variety of routes including oral, rectal, transdermal, subcutaneous, intravenous, intramuscular, and intranasal. Such compositions are useful in treating solid tumors including carcinomas such as ovarian, non-small cell lung, gastric, pancreatic, prostate, renal cell, breast, colorectal, small cell lung, melanoma, and head and neck; and sarcomas such as Kaposi's sarcoma and rhabdomyosarcoma.

The Formula I compounds are preferably administered in the form of oral pharmaceutical compositions. Such compositions are prepared in a manner well known in the pharmaceutical art and comprise at least one active compound.

In another aspect, the present invention also encompasses novel pharmaceutical compositions which contain, as the active ingredient, a compound of Formula I, or a pharmaceutically acceptable salt or solvate thereof, associated with pharmaceutically acceptable carriers, excipients, or diluents. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from 5 to 500 mg, more usually 25 to 300 mg, of the active ingredient. The term "unit dosage form" refers to physically discrete units suitable as unitary dosages dosages for human subjects and other mammals, each unit containing a predetermined quantity of active material calculated to produce the desired therapeutic effect, in association with a suitable pharmaceutical excipient.

The active compounds are effective over a wide dosage range. For examples, dosages per day normally fall within the range of 0.5 to 600 mg/kg of body weight. In the treatment of adult humans, the range of 1 to 50 mg/kg, in single or divided dose, is preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way.

Typical compositions of this invention are described in the following examples:

Formulation Example 1

Hard gelatin capsules containing the following ingredients are prepared:

| Ingredient | Quantity (mg/capsule) |
|---|---|
| N-[[(3,4-dichlorophenyl)amino]carbonyl]-1-methyl-1H-indole-5-sulfonamide | 250.0 |
| Starch | 305.0 |
| Magnesium stearate | 5.0 |

The above ingredients are mixed and filled into hard gelatin capsules in 560 mg quantities.

Formulation Example 2

A tablet formula is prepared using the ingredients below:

| Ingredient | Quantity (mg/tablet) |
|---|---|
| N-[[(4-trifluoromethylphenyl)amino]carbonyl]-2,3-dihydro-1H-indole-5-sulfonamide | 250.0 |
| Cellulose, microcrystalline | 400.0 |
| Colloidal silicon dioxide | 10.0 |
| Stearic acid | 5.0 |

The components are blended and compressed to form tablets, each weighing 665 mg.

Formulation Example 3

A dry powder inhaler formulation is prepared containing the following components:

| Ingredient | Weight % |
|---|---|
| N-[[(3-fluorophenyl)amino]carbonyl]-1-isopropyl-1H-indole-6-sulfonamide | 5 |
| Lactose | 95 |

The active mixture is mixed with the lactose and the mixture is added to a dry powder inhaling appliance.

Formulation Example 4

Tablets, each containing 60 mg of active ingredient, are prepared as follows:

| Ingredient | Quantity (mg/tablet) |
|---|---|
| N-[[(4-bromophenyl)amino]carbonyl]-2,3-dihydro-1H-indole-4-sulfonamide | 60.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 150 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150 mg.

Formulation Example 5

Capsules, each containing 80 mg of medicament are made as follows:

| Ingredient | Quantity (mg/capsule) |
|---|---|
| N-[[(3-(*t*-butyl)phenyl]amino]carbonyl]-2,3-dihydro-1H-indole-5-sulfonamide | 80.0 mg |
| Starch | 109.0 mg |
| Magnesium stearate | 1.0 mg |
| Total | 190.0 mg |

The active ingredient, cellulose, starch, and magnesium stearate are blended, passed through a No. 20 mesh U. S. sieve, and filled into hard gelatin capsules in 190 mg quantities.

Formulation Example 6

Suspensions, each containing 50 mg of medicament per 5.0 ml dose are made as follows:

| Ingredient | Amount |
|---|---|
| N-[[(3,4-dichlorophenyl)amino]carbonyl]-1-ethyl-1H-indole-6-sulfonamide | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor | q.v. |
| Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 7

Suppositories, each containing 225 mg of active ingredient are made as follows:

| Ingredient | Amount |
|---|---|
| N-[[(3-chloro-4-trifluoromethylphenyl)amino]carbonyl]-2,3-dihydro-1H-indole-6-sulfonamide | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 8

Capsules, each containing 150 mg of medicament, are made as follows:

| Ingredient | Quantity (mg/capsule) |
|---|---|
| N-[[(3,4-difluorophenyl)amino]carbonyl]-2,3-dihydro-1H-indole-5-sulfonamide | 150.0 mg |
| Starch | 407.0 mg |
| Magnesium stearate | 3.0 mg |
| Total | 560.0 mg |

The active ingredient, cellulose, starch, and magnesium stearate are blended, passed through a No. 20 mesh U. S. sieve, and filled into hard gelatin capsules in 560 mg quantities.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system

to bypass the blood-brain barrier. One such implantable delivery system, used for the transport of biological factors to specific anatomical regions of the body, is described in U.S. Patent 5,011,472, issued April 30, 1991.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

## Claims

**1.** A compound of the formula

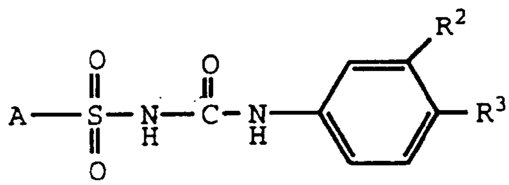

wherein:

A is

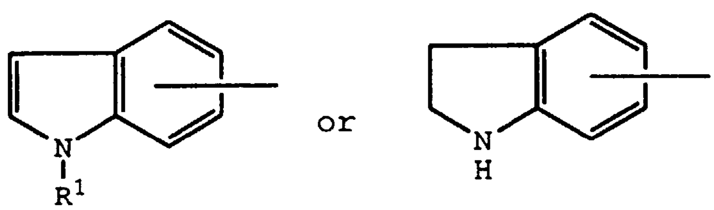

$R^1$ is $C_1$-$C_6$ alkyl;

$R^2$ and $R^3$ are independently selected from the group consisting of hydrogen, halo, $C_1$-$C_6$ alkyl, and trifluoromethyl, provided that no more than one of $R^2$ and $R^3$ can be hydrogen;

or a pharmaceutically acceptable salt or solvate thereof.

**2.** A compound as claimed in Claim 1 wherein A is

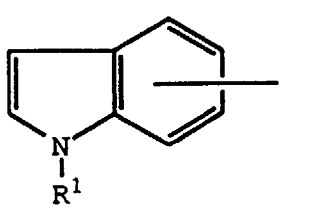

**3.** A compound as claimed in Claim 2 that is N-[[(3,4-dichlorophenyl)amino]carbonyl]-1-methyl-1H-indole-5-sulfonamide or a pharmaceutically acceptable salt or solvate thereof.

**4.** A compound as claimed in Claim 1 wherein A is

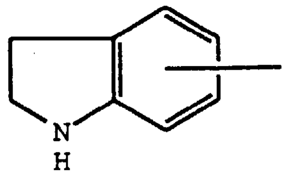

.

5. A compound as as claimed in Claim 4 that is N-[[(3,4-dichlorophenyl)amino]carbonyl]-2,3-dihydro-1H-indole-5-sulfonamide, N-[[(4-chlorophenyl)amino]carbonyl]-2,3-dihydro-1H-indole-5-sulfonamide, or a pharmaceutically acceptable salt or solvate thereof.

6. A pharmaceutical formulation comprising as an active ingredient a compound as claimed in any one of Claims 1 to 5, or a pharmaceutically acceptable salt or solvate thereof, associated with one or more pharmaceutically acceptable carriers, diluents, or excipients therefor.

7. A compound as claimed in any one of Claims 1 to 5, or a pharmaceutically acceptable salt or solvate thereof, for use as an anticancer agent.

8. A process for preparing a sulfonylurea as claimed in any one of Claims 1 to 5, which comprises reacting with a compound of the formula

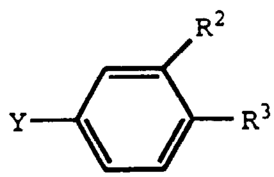

where $R^2$ and $R^3$ are independently selected from the group consisting of hydrogen, halo, $C_1$-$C_6$ alkyl, and trifluoromethyl, provided that no more than one of $R^2$ and $R^3$ can be hydrogen;
and Y is $-NH_2$ or -NCO;
a sulfonyl compound of the formula

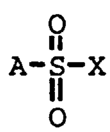

wherein:

A is

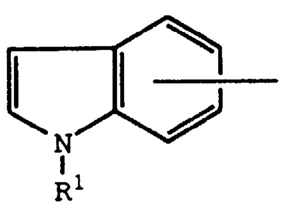

or

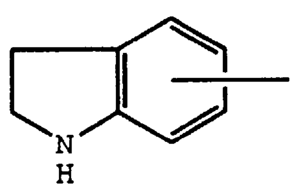

;

$R^1$ is $C_1$-$C_6$ alkyl; and
X is -NCO, $-NH_2$, or $-NH-COOR^a$ in which $R^a$ is $C_1$-$C_3$ alkyl, provided that if X is -NCO or $-NH-COOR^a$, then Y is $-NH_2$, and if X is $-NH_2$, then Y is -NCO.

## Patentansprüche

**1.** Verbindung der Formel

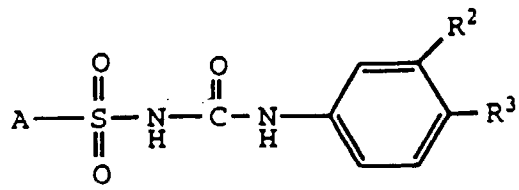

worin

A steht für

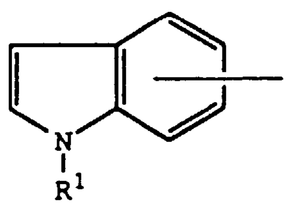

or

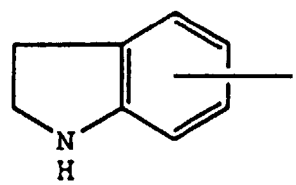

$R^1$ für $C_1$-$C_6$Alkyl steht,
$R^2$ und $R^3$ unabhängig aus der Gruppe ausgewählt sind, die besteht aus Wasserstoff, Halogen, $C_1$-$C_6$Alkyl und Trifluormethyl, mit der Maßgabe, daß nicht mehr als einer von $R^2$ und $R^3$ für Wasserstoff steht,

oder ein pharmazeutisch annehmbares Salz oder Solvat hiervon.

**2.** Verbindung nach Anspruch 1, worin A steht für

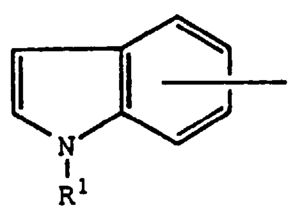

**3.** Verbindung nach Anspruch 2, die N-[(3,4-Dichlorphenyl)amino]carbonyl]-1-methyl-1H-indol-5-sulfonamid oder ein pharmazeutisch annehmbares Salz oder Solvat hiervon ist.

**4.** Verbindung nach Anspruch 1, worin A steht für

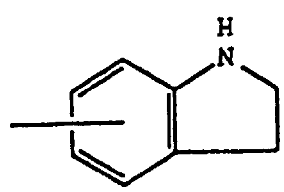

**5.** Verbindung nach Anspruch 4, die N-[(3,4-Dichlorphenyl)amino]carbonyl]-2,3-dihydro-1H-indol-5-sulfonamid, N-[(4-Chlorphenyl)amino]carbonyl]-2,3-dihydro-1H-indol-5-sulfonamid oder ein pharmazeutisch annehmbares Salz oder Solvat hiervon ist.

**6.** Pharmazeutische Formulierung, die als Wirkstoff eine Verbindung nach einem der Ansprüche 1 bis 5 oder ein pharmazeutisch annehmbares Salz oder Solvat hiervon zusammen mit einem oder mehreren pharmazeutisch annehmbaren Trägern, Verdünnungsmitteln oder Hilfsstoffen hierfür enthält.

**7.** Verbindung nach einem der Ansprüche 1 bis 5 oder ein pharmazeutisch annehmbares Salz oder Solvat hiervon zur Verwendung als Antikrebsmittel.

**8.** Verfahren zur Herstellung eines Sulfonylharnstoffes nach einem der Ansprüche 1 bis 5, gekennzeichnet durch die Umsetzung einer Verbindung der Formel

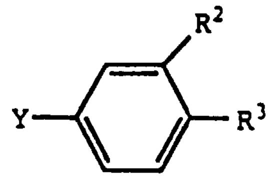

worin

$R^2$ und $R^3$ unabhängig aus der Gruppe ausgewählt sind, die besteht aus Wasserstoff, Halogen, $C_1$-$C_6$Alkyl und Trifluormethyl, mit der Maßgabe, daß nicht mehr als einer von $R^2$ und $R^3$ für Wasserstoff steht,
und Y für $-NH_2$ oder -NCO steht
mit einer Sulfonylverbindung der Formel

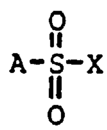

worin

A steht für

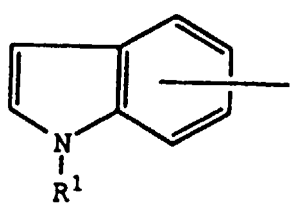

or

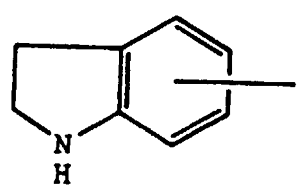

;

$R^1$ für $C_1$-$C_6$Alkyl steht, und
X für -NCO, $-NH_2$ oder -NH-COOR$^a$ steht, worin $R^a$ für $C_1$-$C_3$Alkyl steht, mit der Maßgabe, daß falls X für -NCO oder -NH-COOR$^a$ steht, Y dann für $-NH_2$ steht und falls X für $-NH_2$ steht, Y dann für -NCO steht.

## Revendications

**1.** Composé répondant à la Formule

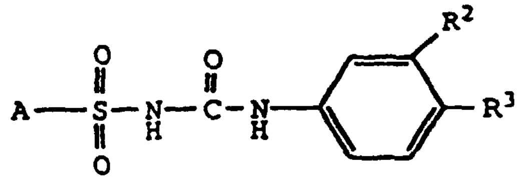

dans laquelle :

A représente

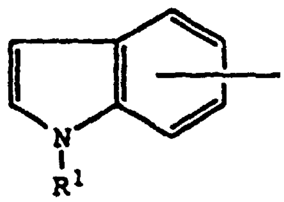

ou

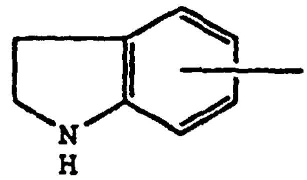

$R^1$ représente un groupe alkyle en $C_1$-$C_6$;
$R^2$ et $R^3$ sont choisis indépendamment l'un de l'autre parmi le groupe constitué d'un atome d'hydrogène, d'un atome d'halogène, d'un groupe alkyle en $C_1$-$C_6$, et d'un groupe trifluorométhyle, sans que plus d'un groupe parmi $R^2$ et $R^3$ ne puisse être un atome d'hydrogène;

ou un sel ou un solvate pharmaceutiquement acceptable de celui-ci.

2. Composé selon la revendication 1, dans lequel A représente

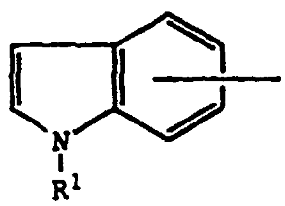

.

3. Composé selon la revendication 2, qui est le N-[[(3,4-dichlorophényl)amino]carbonyl]-1-méthyl-1H-indole-5-sulfonamide ou un sel ou un solvate pharmaceutiquement acceptable de celui-ci.

4. Composé selon la revendication 1, dans lequel A représente

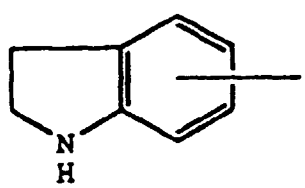

.

5. Composé selon la revendication 4, qui est le N-[[(3,4-dichlorophényl)amino]carbonyl]-2, 3-dihydro-1H-indole-5-sulfonamide, le N-[[(4-chlorophényl)amino]carbonyl]-2,3-dihydro-1H-indole-5-sulfonamide ou un sel ou un solvate pharmaceutiquement acceptable de ceux-ci.

6. Formulation pharmaceutique comprenant, à titre d'ingrédient actif, un composé selon l'une quelconque des revendications 1 à 5, ou un sel ou un solvate pharmaceutiquement acceptable de celui-ci, associé à un ou à plusieurs supports, diluants ou excipients pharmaceutiquement acceptables pour celui-ci.

**7.** Composé selon l'une quelconque des revendications 1 à 5, ou un sel ou un solvate pharmaceutiquement acceptable de celui-ci, pour l'utilisation comme agent contre le cancer.

**8.** Procédé de préparation d'une sulfonylurée selon l'une quelconque des revendications 1 à 5, qui comprend la mise en réaction avec un composé répondant à la formule

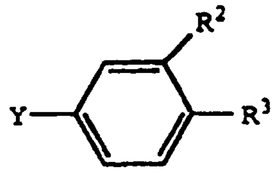

où $R^2$ et $R^3$ sont choisis indépendamment l'un de l'autre parmi le groupe constitué d'un atome d'hydrogène, d'un atome d'halogène, d'un groupe alkyle en $C_1$-$C_6$, et d'un groupe trifluorométhyle, sans que plus d'un groupe parmi $R^2$ et $R^3$ ne puisse être un atome d'hydrogène;
et Y représente $-NH_2$ ou -NCO;
d'un composé sulfonyle répondant à la formule

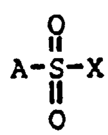

dans laquelle :

A représente

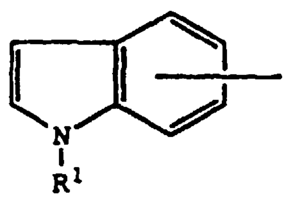

ou

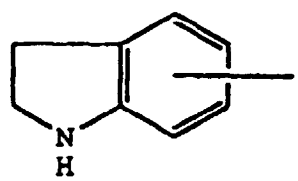

;

$R^1$ représente un groupe alkyle en $C_1$-$C_6$, et
X représente -NCO, $-NH_2$, ou $-NH-COOR^a$, formule dans laquelle $R^a$ représente un groupe alkyle en $C_1$-$C_3$, pour autant que si X représente -NCO ou $-NH-COOR^a$, alors Y représente $-NH_2$, et que si X représente $-NH_2$, alors Y représente -NCO.